(19) Europäisches Patentamt
European Patent Office
Office européen des brevets

(11) Publication number: **0 353 678 B1**

(12) # EUROPEAN PATENT SPECIFICATION

(45) Date of publication of patent specification: **13.12.95**

(51) Int. Cl.[6]: **C12P 19/44**, C07H 19/01

(21) Application number: **89114071.7**

(22) Date of filing: **29.07.89**

(54) **FR-901154 and FR-901155 substances, a process for their production**

(30) Priority: **01.08.88 GB 8818259**
**01.11.88 GB 8825517**

(43) Date of publication of application:
**07.02.90 Bulletin 90/06**

(45) Publication of the grant of the patent:
**13.12.95 Bulletin 95/50**

(84) Designated Contracting States:
**AT BE CH DE ES FR GB GR IT LI LU NL SE**

(56) References cited:
**EP-A- 0 184 162**
**EP-A- 0 323 042**
**EP-A- 0 349 049**

(73) Proprietor: **FUJISAWA PHARMACEUTICAL CO., LTD.**
**4-7, Doshomachi 3-chome**
**Chuo-ku**
**Osaka-shi**
**Osaka 541 (JP)**

(72) Inventor: **Okuhara, Masakuni**
**14-10, Umezono 2-chome**
**Tsukuba-shi**
**Ibaraki 305 (JP)**
Inventor: **Goto, Toshio**
**1-14-20, Sengen**
**Tsukuba-shi**
**Ibaraki 305 (JP)**
Inventor: **Hatanaka, Hiroshi**
**4-15-1-205, Namiki**
**Tsukuba-shi**
**Ibaraki 305 (JP)**
Inventor: **Yuasa, Seiichi 72-7, Aza-Icchobata**
**Oaza-Ifuku**
**Shippocho**
**Ama-gun**
**Aichi 497 (JP)**
Inventor: **Nakatani, Isami**
**806-1, Egiracho**
**Hashima-shi**
**Gifu 501-62 (JP)**

(74) Representative: **Türk, Gille, Hrabal, Leifert**
**Brucknerstrasse 20**
**D-40593 Düsseldorf (DE)**

EP 0 353 678 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel processes for preparing compounds having pharmacological activities, hereinafter entitled FR-901154 and FR-901155 substances.

FR-901154 and FR-901155 are substances which have pharmacological activities such as inmunosuppressive activity, antimicrobial activity.

Accordingly, the object of this invention is to provide novel processes for preparing the compounds, FR-901154 and FR-901155 substances which are useful for treatment and prevention of rejection by transplantation, graft-versus-host diseases by medulla ossium transplantation, autoimmune diseases, infectious diseases, and the like.

Another object of this invention is to provide a process for production of the FR-901154 and FR-901155 substances by fermentation of a FR-901154 and/or FR-901155 substance(s)-producing strain belonging to the genus Streptomyces in a nutrient medium.

And further, the present invention relates to a novel process for the production of the FR-901156 substance.

Accordingly, the other object of this invention is to provide a process for production of the FR-901156 substance by fermentation of a FR-901156-producing strain belonging to the genus Streptomyces in a nutrient medium.

Earlier European patent application EP-A-0 349 049 discloses a macrolide designated L-682,993 having a structure identical to the compound FR-901154, which is prepared by fermentation of Actinoplanacete sp., ATCC No. 53771.

A process for preparing FR-901156 by chemical modification of another macrocylic compound is described in European patent application EP-A-0 323 042.

A similar process for preparing FR-901 156 by chemical modification is described in EP-A-0 184 162, which discloses the newly discovered FR-900506 substance [chemical name: 17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo-[22.3.1.$0^{4,9}$]octacos-18-ene-2,3,10,16-tetraone] which was firstly and newly isolated in pure form from culture broth obtained by fermentation of new species belonging to genus Streptomyces and is of use as medicament.

Based on the discovery of the FR-900506 substance, the inventors of the present invention studied to produce the FR-900506 substance by fermentation in high yield, and during its study they have found that the FR-901154 and FR-901155 substances were also produced in the culture broth.

The physical and chemical properties of the FR-901154 and FR-901155 substances of the present invention, their production and their pharmacological activities are explained hereinbelow.

### PHYSICAL AND CHEMICAL PROPERTIES OF FR-901154 AND FR-901155 SUBSTANCES

The FR-901154 and FR-901155 substances of the present invention possess the physical and chemical properties, as defined in claim 1.

With regard to the FR-901154 and FR-901155 substances, it is to be noted that in case of measurements of $^{13}C$ and $^{1}H$ nuclear magnetic resonance spectra, this substance showed pairs of the signals on the various chemical shifts, but in case of the measurements of the thin layer chromatography and the high performance liquid chromatography, the FR-901154 and FR-901155 substances showed a single spot in the thin layer chromatography and a single peak in the high performance liquid chromatography, respectively.

From the above physical and chemical properties, the FR-901154 substance is inferred to have the following chemical structure.

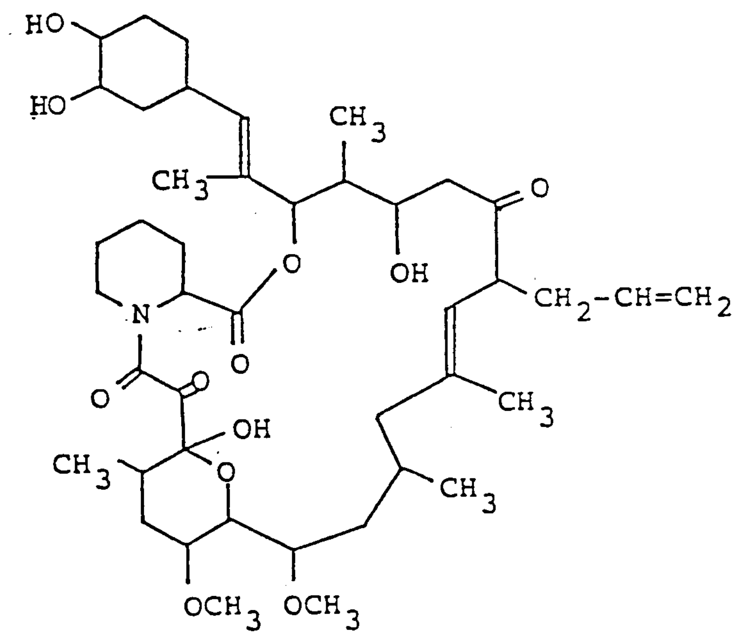

From the above physical and chemical properties, the FR-901155 substance is inferred to have the following chemical structure.

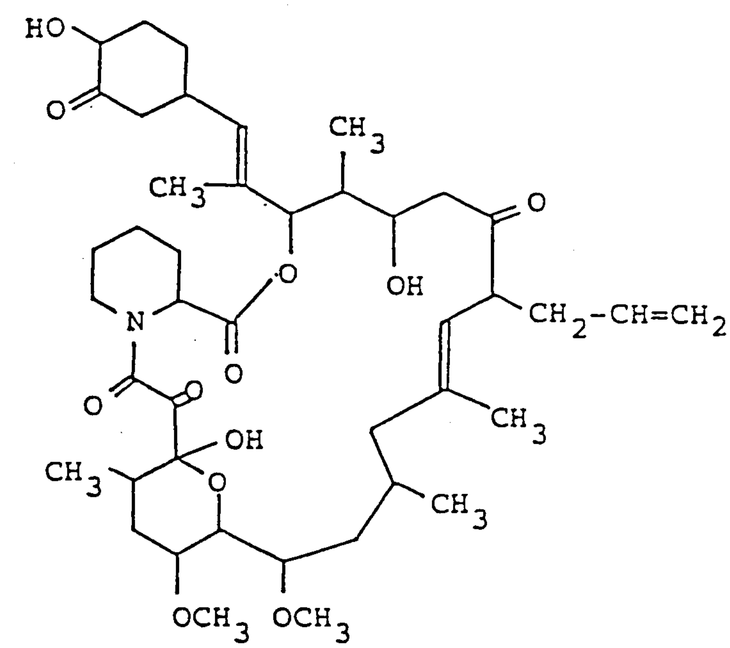

PRODUCTION OF FR-901154 AND FR-901155 SUBSTANCES

The FR-901154 and FR-901155 substances of this invention can be produced by fermentation of a FR-901154 and/or FR-901155 substance(s)-producing strain belonging to the genus Streptomyces such as Streptomyces tsukubaensis No. 9993 in a nutrient medium.

A lyophilized sample of the Streptomyces tsukubaensis No. 9993 was deposited with the Fermentation Research Institute, Agency of Industrial Science and Technology (No. 1-3, Higashi 1-chome, Tsukuba-shi, Ibaraki Prefecture, Japan) under the number of FERM P-7886 (deposited date: October 5th, 1984), and was converted to the Budapest Treaty route of the same depository on October 19, 1985 under the new deposit

number of FERM BP-927.

It is to be understood that the production of the FR-901154 and FR-901155 substances is not limited to the use of the particular organism described above, which is given for the illustrative purpose only. This invention also includes the use of any mutants which are capable of producing the FR-901154 and/or FR-901155 substances including natural mutants as well as artificial mutants which can, be produced from the described organism by conventional means such as irradiation of X-rays, ultra-violet radiation, treatment with N-methyl-N'-nitro-N-nitrosoguanidine, 2-aminopurine, and the like.

In general, the FR-901154 and FR-901155 substances can be produced by culturing the FR-901154 and/or FR-901155 substance(s)-producing strain in an aqueous nutrient medium containing sources of assimilable carbon and nitrogen, preferably under aerobic conditions (e.g. shaking culture, submerged culture, etc.).

The preferred sources of carbon in the nutrient medium are carbohydrates such as glucose, xylose, galactose, glycerin, starch, dextrin, and the like. Other sources which may be included are maltose, rhamnose, raffinose, arabinose, mannose, salicin, sodium succinate, and the like.

The preferred sources of nitrogen are yeast extract, peptone, gluten meal, cottonseed meal, soybean meal, corn steep liquor, dried yeast, wheat germ, feather meal, peanut powder etc., as well as inorganic and organic nitrogen compounds such as ammonium salts (e.g. ammonium nitrate, ammonium sulfate, ammonium phosphate, etc.), urea, amino acid, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salt and the like. If necessary, especially when the culture medium foams seriously, a defoaming agent, such as liquid paraffin, fatty oil, plant oil, mineral oil or silicone may be added.

As the conditions for the production of the FR-901154 and FR-901155 substances in massive amounts, submerged aerobic cultural conditions are preferred therefor. For the production in small amounts, a shaking or surface culture in a flask or bottle is employed. Furthermore, when the growth is carried out in large tanks, it is preferable to use the vegetative form of the organism for inoculation in the production tanks in order to avoid growth lag in the process of production of the FR-901154 and FR-901155 substances. Accordingly, it is desirable first to produce a vegetative inoculum of the organism by inoculating a relatively small quantity of culture medium with spores or mycelia of the organism and culturing said inoculated medium, and then to transfer the cultured vegetative inoculum aseptically to large tanks. The medium, in which the vegetative inoculum is produced, is substantially the same as or different from the medium utilized for the production of the FR-901154 and FR-901155 substances.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The fermentation is usually conducted at a temperature between about 20°C and 40°C, preferably 25-35°C, for a period of about 50 hours to 250 hours, preferably 100 hours to 200 hours which may be varied according to fermentation conditions and scales.

Thus produced FR-901154 and FR-901155 substances can be recovered from the culture medium by conventional means which are commonly used for the recovery of other known biologically active substances. The FR-901154 and FR-901155 substances produced are found in the cultured mycelium and filtrate, and accordingly the FR-901154 and FR-901155 substances can be isolated and purified from the mycelium and the filtrate, which are obtained by filtering or centrifuging the cultured broth, by a conventional method such as concentration under reduced pressure, lyophilization, extraction with a conventional solvent, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like.

## PHARMACOLOGICAL ACTIVITIES OF THE FR-901154 AND FR-901155 SUBSTANCES

The FR-901154 and FR-901155 substances possess pharmacological activities such as immunosuppressive activity, antimicrobial activity, and the like, and therefore are useful for the treatment and prevention of the rejection by transplantation of organs or tissues such as heart, kidney, liver, medulla ossium, skin, cornea etc., graft-versus-host diseases by medulla ossium transplantation, autoimmune

diseases such as rheumatoid arthritis, systemic lupus erythematosus, Hashimoto's thyroiditis, multiple sclerosis, myasthenia gravis, type I diabetes, uveitis such as Behcet's disease, etc., vernal keratoconjunctivitis, infectious diseases caused by pathogenic microorganisms, and the like.

And further, the FR-901154 and FR-901155 substances are also useful in the topical administration for the treatment and the prophylaxis of inflammatory and hyperproliferative skin diseases and cutaneous manifestations of immunologically-mediated illnesses, such as, psoriasis, atopical dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and Alopecia areata.

As examples for showing such pharmacological activities, some pharmacological test data of the FR-901154 and FR-901155 substances are illustrated in the following.

Test 1

Suppression of in vitro Mixed Lymphocyte Reaction (MLR)

The MLR test was performed in microtiter plates, with each well containing 5 x $10^5$ C57BL/6 responder cells (H-$2^b$), 5 x $10^5$ mitomycin C treated (25μg/ml mitomycin C at 37 °C for 30 minutes and washed three times with RPMI 1640 medium) BALB/C stimulator cells (H-$2^d$) in 0.2 ml RPMI 1640 medium supplemented with 10% fetal calf serum, 2mM sodium bicarbonate, penicillin (50 unit/ml) and streptomycin (50 μg/ml). The cells were incubated at 37 °C in humidified atmosphere of 5% carbon dioxide and 95% of air for 68 hours and pulsed with $^3$H-thymidine (0.5 μCi) 4 hours before the cells were collected. The object compound of this invention were dissolved in ethanol and further diluted in RPMI 1640 medium and added to the cultures to give final concentrations of 100nM or less.

The $IC_{50}$ values (mol concentration to suppress 50% of MLR) were calculated by a conventional method, which are shown in the following Table 1.

Table 1

| $IC_{50}$ Values of MLR test on FR-901154 and FR-901155 Substances | |
|---|---|
| Substances | $IC_{50}$ Values (mol/liter) |
| FR-901154 | $6.1 \times 10^{-10}$ |
| FR-901155 | $7.1 \times 10^{-10}$ |

Test 2

Antimicrobial activities of the FR-901154 and FR-901155 Substances

Antimicrobial activities of the FR-901154 and FR-901155 substances against various fungi were determined by a serial agar dilution method in a Sabouraud agar. Minimum inhibitory concentrations (MIC) were expressed in terms of μg/ml after incubation at 30 °C for 24 hours.

Both of the FR-901154 and FR-901155 substances showed antimicrobial activities against fungi, for example, Aspergillus niger IFO 4417 as described in the following.

Table 2

| MIC values (μg/ml) of FR-901154 and FR-901155 Substances | |
|---|---|
| Substances | MIC Value (μg/ml) |
| FR-901154 | 0.025 |
| FR-901155 | 0.013 |

The pharmaceutical composition of this invention can be used in the form of a pharmaceutical preparation, for example, in solid, semisolid or liquid form, which contains the FR-901154 or FR-901155 substance, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, injections, ointments, liniments, eye drops lotion, gel, creme and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening, solubilizing and coloring agents and perfumes may be used. Particularly, as a sulubilizing agent, there may be exemplified water-soluble cellulose polymer ( i.e. hydroxypropyl methyl-cellulose, etc.), water-soluble glycol (i.e. propylene glycol, etc.), etc. The active object compound is included in the pharmaceutical composition in an amount sufficient to produce the desired effect upon the process or condition of diseases.

For applying this composition to human, it is preferable to apply it by parenteral or enteral administration. While the dosage of therapeutically effective amount of the FR-901154 or FR-901155 substance varies from and also depends upon the age and condition of each individual patient to be treated, a daily dose of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

And further, the present invention relates to a novel process for the production of the FR-901156 substance.

Accordingly, the other object of this invention is to provide a process for production of the FR-901156 substance by fermentation of a FR-901156-producing strain belonging to the genus Streptomyces in a nutrient medium.

The fermentation of the FR-901156 substance-producing strain belonging to the genus Streptomyces, and the isolation and purification of the FR-901156 substance can be carried out in a similar manner to those of the FR-901154 and/or the FR-901155 substance(s).

The following examples are given for the purpose of illustrating the present invention.

## Example 1

### Fermentation

A culture medium (100 ml) containing corn starch (1%), glycerin (1%), glucose (0.5%), cottonseed meal (1%), dried yeast (0.5%), corn steep liquor (0.5%) and calcium carbonate (0.2%) (adjusted to pH 6.5) was poured into each of eight 500 ml-Erlenmeyer flasks and sterilized at 120°C for 30 minutes. A loopful of slant culture of Streptomyces tsukubaensis No.9993, FERM P-7886 was inoculated to each of the media and cultured at 30°C for 72 hours on a rotary shaker. The seed culture was transferred to the same seed medium (160 liters) added Adekanol (defoaming agent, Trade Mark, maker; Asahi Denka Co.) (0.05%) and Silicone (Shinetsu Chemical Co.) (0.05%), in a 200 liter jar-fermentor which had been sterilized at 120°C for 30 minutes in advance. After the culture was performed at 30°C for 48 hours under aeration of 160 liters/minute and agitation at 200 rpm, 30 liters of the preculture was inoculated to a production medium (3000 liters) containing soluble starch (3%), wheat germ (0.8%), dried yeast (0.4%), corn steep liquor (0.6%), calcium carbonate (0.1%), Adekanol (0.05%) and Silicone (0.05%) at pH 6.8 in a 4 ton tank, which had been sterilized at 120°C for 30 minutes in advance. The fermentation was carried out at 25°C for 168 hours under aeration of 1500 liters/minute and agitation at 140 rpm.

### Isolation and Purification

The cultured broth thus obtained was filtered with an aid of diatomaceous earth (50 kg). The mycelial cake was extracted with acetone (1000 liters), yielding 1000 liters of the extract. The acetone extract from mycelium and the filtrate (2700 liters) were combined and passed through a column of a non-ionic adsorption resin "Diaion HP-20" (Trade Mark, maker Mitsubishi Chemical Industries Ltd.) ( 200 liters). After washing with 50% aqueous acetone (600 liters), elution was carried out with 75% aqueous acetone. The eluate was evaporated under reduced pressure to give residual water (40 liters). This residue was extracted with ethyl acetate (40 liters) twice. The ethyl acetate extract was concentrated under reduced pressure to give an oily residue. The oily residue was dissolved in a mixture of n-hexane and ethyl acetate (1:1 v/v, 3

liters) and subjected to column chromatography on silica gel (maker: Merck & Co., Ltd. 70-230 mesh) (70 liters) packed with the same solvent system.

Elution was carried out with a mixture of n-hexane and ethyl acetate (1:1 v/v, 420 liters and 1:2 v/v, 420 liters), ethyl acetate (210 liters) and acetone (210 liters). Fractions were collected at elution volume from 350 liters to 420 liters (the first eluate), 490 liters to 840 liters (the second eluate) and 980 liters to 1190 liters (the third eluate), respectively.

The third eluate was evaporated to dryness in vacuo. The resultant product was dissolved in a mixture of chloroform and acetonitrile (2:1 v/v, 400 ml) and subjected to column chromatography on silica gel (maker; Merck & Co., 230 - 400 mesh) (2 liters). Fractions containing the desired compound were collected and evaporated in vacuo to give 6.8 g of the purified FR-901154 substance in a form of white powder.

The first eluate was evaporated to dryness in vacuo. The resultant product was dissolved in a mixture of n-hexane and ethyl acetate (1:2.5 v/v, 25 ml) and subjected to column chromatography on silica gel (230 - 400 mesh) (750 ml) packed and developed with the same solvent. Fractions containing the desired compound were collected and evaporated in vacuo to give a crude powder, which was rechromatographed on the same silica gel (50 ml). Fractions containing the desired compound were collected and evaporated to dryness in vacuo to give 390 mg of the purified FR-901155 substance in a form of white powder.

The second eluate was evaporated to dryness in vacuo. The resultant product was dissolved in a mixture of n-hexane and ethyl acetate (1:1 v/v, 5 liters) and subjected to column chromatography on silica gel (70 - 230 mesh) (70 liters) mixed with silver nitrate (20% w/w). After washing with the same solvent (350 liters), elution was carried out with a mixture of n-hexane and ethyl acetate (1:2 v/v, 490 liters). Fractions containing the desired compound were collected and evaporated in vacuo to give a crude powder. This powder was dissolved in ethyl acetate (100 ml) and then chromatographed on silica gel (230 - 400 mesh) (1000 ml) with the same solvent. Active fractions were collected and evaporated to dryness in vacuo. The residue was crystallized from a mixture of acetonitrile and water (1:2 v/v, 30 ml). This crystal was dissolved in 60% aqueous acetonitrile (10 ml) and chromatographed on "Diaion CHP-20" (Trade Mark, maker: Mitsubishi Chemical Industries Ltd.) (100 ml). After evaporation, the residue was recrystallized to give 270 mg of the purified FR-901156 substance as colorless prisms.

Physical and chemical properties of the FR-901156 substance

(1) Mass Spectrum:
FAB-MS: m/z 828 (M + Na)

(2) Molecular Formula:

$C_{44}H_{71}NO_{12}$

(3) Elemental Analysis for $C_{44}H_{71}NO_{12}.H_2O$:

| | C | H | N |
|---|---|---|---|
| Found<br>Calcd. | 63.93 %,<br>64.13 % | 8.55 %,<br>8.93 % | 1.70 %<br>1.70 % |

(4) Color Reaction:
Positive: cerium sulfate reaction, sulfuric acid reaction, Ehrlich reaction, Dragendorff reaction and iodine vapor reaction
Negative: ferric chloride reaction, ninhydrin reaction and Molish reaction

(5) Solubility:
Soluble : methanol, ethanol, acetone, ethyl acetate, chloroform, diethyl ether, benzene and acetonitrile
Sparingly Soluble: n-hexane and petroleum ether
Insoluble: water

(6) Melting Point:
146 - 147 °C

(7) Specific Rotation:
$[\alpha]_D^{23}$ : -74° (c = 1.8, $CHCl_3$)

(8) Ultraviolet Absorption Spectrum:
end absorption

(9) Infrared Absorption Spectrum:

$$\nu_{max}^{CHCl_3}:$$

3570, 3500, 2940, 2880, 2820, 1740, 1710, 1700, 1640, 1460, 1450, 1380, 1350, 1320, 1280, 1230, 1190, 1170, 1140, 1100, 1090, 1050, 1040, 1000, 990, 940, 910 $cm^{-1}$

(10) $^{13}C$ Nuclear Magnetic Resonance Spectrum:

δ(ppm, $CDCl_3$, 100MHz):

| | | |
|---|---|---|
| {213.24(s) / 213.32(s), | {196.06(s) / 192.70(s), | {168.91(s) / 168.62(s), |
| {164.61(s) / 165.68(s), | {138.33(s) / 139.19(s), | {132.12(s) / 131.66(s), |
| {129.68(d) / 129.52(d), | {123.19(d) / 123.43(d), | {96.98(s) / 98.51(s), |
| 84.04(d), | {77.32(d) / 77.78(d), | {75.12(d) / 76.54(d), |
| {73.56(d) / 73.50(d), | {73.40(d) / 73.38(d), | {72.75(d) / 72.13(d), |
| {69.89(d) / 68.95(d), | {56.85(q) / 57.40(q), | {56.48(d) / 52.58(d), |
| {56.46(q) / 56.42(q), | {56.16(q) / 55.94(q), | 52.87(d), |
| {48.54(t) / 48.31(t), | {43.20(t) / 43.42(t), | {39.62(d) / 40.17(d), |
| {39.11(t) / 43.77(t), | {34.74(d) / 34.77(d), | {34.74(t) / 34.64(t), |
| {34.46(d) / 33.51(d), | {33.19(t) / 33.37(t), | {32.90(t) / 35.29(t), |
| {32.54(t) / 32.39(t), | 31.12(t), | 30.49(t), |

| | | |
|---|---|---|
| {27.44(t)<br>{26.07(t), | {26.19(d)<br>{25.90(d), | {24.33(t)<br>{24.45(t), |
| {20.95(t)<br>{20.69(t), | {20.32(q)<br>{19.40(q), | {20.27(t)<br>{20.21(t), |
| {16.06(q)<br>{15.85(q), | {15.71(q)<br>{15.56(q), | {13.96(q)<br>{13.82(q), |
| {13.92(q)<br>{14.09(q), | {9.36(q)<br>{9.67(q), | |

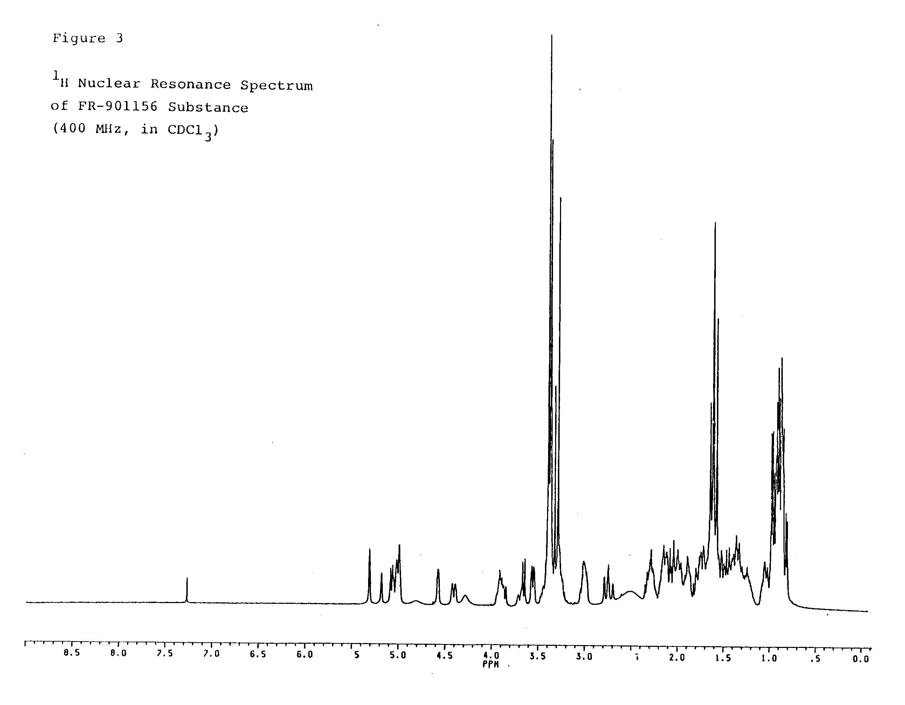

(11) $^1H$ Nuclear Magnetic Resonance Spectrum:
the chart of which being shown in Figure 3,
(12) Thin Layer Chromatography:

| Stationary Phase | Developing Solvent | Rf Values |
|---|---|---|
| silica gel plate | ethyl acetate | 0.51 |

(13) Property of the Substance:
neutral substance

From the above physical and chemical properties, the FR-901156 substance is inferred to be 1,14-dihydroxy-12-[2-(4-hydroxy-3-methoxycyclohexyl)-1-methylvinyl]-23,25-dimethoxy-13,19,21,27-tetramethyl-17-propyl-11,28-dioxa-4-azatricyclo[22.3.1.$0^{4,9}$]octacos-18-ene-2,3,10,16-tetraone.

And by the following procedures, the object compounds of the present invention, i.e. the FR-901154 and FR-901155 substances, are also isolated and purified, and furthermore separated from the other compounds, e.g., the FR-901156, FR-900506 and FR-900520 substances.

Isolation and Purification

The cultured broth obtained in a before-mentioned manner was filtered with an aid of diatomaceous earth (50 kg). The mycelial cake was extracted with acetone (1000 liters), yielding 1000 liters of the extract. The acetone extract from the mycelium and the filtrate (2700 liters) were combined and passed through a column of non-ionic adsorption resin "Diaion HP-20" (200 liters). After washing with 50% aqueous acetone (600 liters), elution was carried out with 75% aqueous acetone. The eluate was evaporated under reduced pressure to give aqueous residue (600 liters). This residue were passed through a column containing 150 liters of activated carbon (Granular, maker; Takeda Pharmaceutical Co., Ltd.). After washing with 50% aqueous acetone (200 liters), elution was carried out with ethyl acetate (400 liters) as eluant. The ethyl acetate eluate was concentrated under reduced pressure to give an oily residue. The oily residue was dissolved in a mixture of n-hexane and ethyl acetate (2:1 v/v, 20 liters) and subjected to column chromatography on silica gel (70-230 mesh)(70 liters) packed with the same solvent system.

Elution was carried out with following: a mixture of n-hexane and ethyl acetate (1:1 v/v, 140 liters and 1:2 v/v, 420 liters), ethyl acetate (140 liters) and methyl alcohol (100 liters). Fractions were collected at elution volume from 250 liters to 550 liters (the first eluate), 700 liters to 800 liters (the second eluate), respectively.

The second eluate was evaporated to dryness in vacuo. The resultant product was dissolved in a mixture of chloroform and acetonitrile (2:1 v/v, 400 ml) and subjected to column chromatography on silica gel (230-400 mesh) (2 liters). Fractions containing the desired compound were collected and evaporated in vacuo to give 9.3 g of the purified FR-901154 substance in a form of white powder.

The first eluate was evaporated to dryness in vacuo. The resultant product was dissolved in a mixture of n-hexane, acetone and methyl alcohol (40:20:1 v/v/v, 20 liters) and subjected to column chromatography on silica gel (70-230 mesh) (70 liters) mixed with silver nitrate (20% w/w) and developed with the same solvent (400 liters). Fractions were collected at elution volume from 160 liters to 190 liters (the third eluate), 190 liters to 220 liters (the fourth eluate) and 250 liters to 400 liters (the fifth eluate), respectively.

The third eluate was evaporated to dryness in vacuo. The resultant product was dissolved in ethyl acetate (100 ml) and then chromatographed on silica gel (230-400 mesh) (1 liter) with the same solvent. Active fractions were collected and evaporated to dryness in vacuo. The residue was crystallized from a mixture of acetonitrile and water (1:2 v/v, 30 ml). This crystal was dissolved in 60% aqueous acetonitrile (10 ml) and chromatographed on "Diaion CHP-20P" (Trade Mark, maker; Mitsubishi Chemical Industries Ltd.)-(100 ml). After evaporation, the residue was recrystallized to give 400 mg of the purified FR-901156 substance as colorless prisms.

The fourth eluate was concentrated under reduced pressure to give an oily residue. The oily residue was dissolved in methyl alcohol (5 ml) and subjected to column chromatography on "octadecyl C-18 gel" (Trade Mark, maker; Yamamura Chemical Lab. Co., Ltd.)(60-200 mesh) (800 ml). The elution was carried out with 80% aqueous methyl alcohol. Fractions containing the desired compound were collected and evaporated under reduced pressure to give residual water. This residue was extracted with ethyl acetate (400 ml). The ethyl acetate extract was evaporated to dryness in vacuo. The resultant product was crystallized from a mixture of acetonitrile and water (1:2 v/v, 9 ml) to give 390 mg of the purified FR-900520 substance as colorless prisms.

The fifth eluate was concentrated under reduced pressure to give an oily residue. The oily residue was crystallized from a mixture of ethyl alcohol and water (1:2 v/v, 900 ml). This crystal was recrystallized with ethyl acetate to give 80 g of the purified FR-900506 substance as colorless prisms.

The mother liquor was evaporated to dryness in vacuo. The resultant product was dissolved in a mixture of n-hexane and ethyl acetate (1:2 v/v, 500 ml) and subjected to column chromatography on silica gel (70-230 mesh)(7 liters). Elution was carried out with a mixture of n-hexane and ethyl acetate (1:2 v/v, 2.5 liters). Fractions containing the desired compound were collected and evaporated to dryness in vacuo to give a crude powder, which was rechromatographed on the same silica gel (100 ml). Fractions containing the desired compound were collected and evaporated to dryness in vacuo to give 750 mg of the purified FR-901155 substance in a form of white powder.

Brief Description of Drawings

Figure 1 shows $^1H$ Nuclear Magnetic Resonance Spectrum of the FR-901154 substance, Figure 2 shows $^1H$ Nuclear Magnetic Resonance Spectrum of the FR-901155 substance and Figure 3 shows $^1H$ Nuclear Magnetic Resonance Spectrum of the FR-901156 substance.

**Claims**

**1.** A process for preparing a compound selected from FR-901154 substance and FR-901155 substance, wherein

1) FR-901154 Substance having the following physical and chemical properties;

(1) Form and Color:
white powder

(2) Mass Spectrum:
FAB-MS: m/z 812 (M + Na)

(3) Molecular Formula:

$C_{43}H_{67}NO_{12}$

(4) Elemental Analysis for $C_{43}H_{67}NO_{12}.H_2O$:

| | C | H | N |
|---|---|---|---|
| Found | 63.80 %, | 8.81 %, | 1.79 % |
| Calcd. | 63.92 % | 8.61 % | 1.73 % |

(5) Color Reaction:

Positive: cerium sulfate reaction, sulfuric acid reaction, Ehrlich reaction, Dragendorff reaction and iodine vapor reaction

Negative: ferric chloride reaction, ninhydrin reaction and Molish reaction

(6) Solubility:

Soluble : methanol, ethanol, acetone, ethyl acetate, chloroform, diethyl ether,

| | |
|---|---|
| | benzene and acetonitrile |
| Sparingly Soluble: | n-hexane and petroleum ether |
| Insoluble: | water |

(7) Melting Point:
96 - 97 °C
(8) Specific Rotation:
$[\alpha]_D^{23}$ : -65° (c = 1.7, $CHCl_3$)
(9) Ultraviolet Absorption Spectrum:
end absorption
(10) Infrared Absorption Spectrum:

$$\nu_{max}^{CHCl_3}:$$

3570, 3500, 2920, 2860, 2820, 1740, 1710, 1700, 1640, 1460, 1450, 1380, 1340, 1280, 1210, 1190, 1170, 1140, 1100, 1090, 1050, 1010, 1000, 990, 960, 920 $cm^{-1}$
(11) $^{13}C$ Nuclear Magnetic Resonance Spectrum:
$\delta$(ppm, $CDCl_3$, 100MHz):

| | | |
|---|---|---|
| 212.51 (s)<br>212.44 (s), | 196.28 (s)<br>192.78 (s), | 168.98 (s)<br>168.71 (s), |
| 164.72 (s)<br>165.84 (s), | 138.89 (s)<br>139.64 (s), | 135.46 (d)<br>135.32 (d), |
| 132.21 (s)<br>131.78 (s), | 129.93 (d)<br>129.50 (d), | 122.42 (d)<br>122.60 (d), |
| 116.58 (t), | 97.00 (s)<br>98.52 (s), | 77.47 (d)<br>77.76 (d), |
| 75.31 (d), | 75.13 (d)<br>76.58 (d), | 74.73 (d)<br>74.77 (d), |
| 73.59 (d)<br>73.56 (d), | 72.76 (d)<br>72.18 (d), | 69.87 (d)<br>69.02 (d), |
| 56.92 (q)<br>57.45 (q), | 56.24 (q)<br>56.03 (q), | 48.55 (t)<br>48.27 (t), |
| 43.49 (t)<br>43.86 (t), | 39.75 (d)<br>40.22 (d), | 39.18 (t)<br>43.86 (t), |
| 38.95 (t), | 35.19 (t)<br>35.47 (t), | 34.90 (d)<br>34.93 (d), |
| 34.54 (d)<br>33.54 (d), | 32.96 (t)<br>35.40 (t), | 32.60 (t)<br>32.46 (t), |
| 31.94 (t)<br>31.89 (t), | 30.83 (t), | 27.58 (t), |
| 26.16 (d)<br>26.02 (d), | 24.39 (t)<br>24.48 (t), | 21.02 (t)<br>20.74 (t), |
| 20.31 (q)<br>19.41 (q), | 16.14 (q)<br>15.91 (q), | 15.84 (q)<br>15.81 (q), |
| 13.87 (q)<br>14.17 (q), | 9.41 (q)<br>9.73 (q), | |

(12) $^{1}H$ Nuclear Magnetic Resonance Spectrum:
the chart of which being shown in Figure 1,
(13) Thin Layer Chromatography:

| Stationary Phase | Developing Solvent | Rf Values |
|---|---|---|
| silica gel plate | ethyl acetate | 0.22 |

(14) Property of the Substance:
neutral substance
and
2)FR-901155 Substance having the following physical and chemical properties;
(1) Form and Color:
white powder

(2) Mass Spectrum:
FAB-MS: m/z 810 (M + Na)
(3) Molecular Formula:

$C_{43}H_{65}NO_{12}$

(4) Elemental Analysis for $C_{43}H_{65}NO_{12}$:

| | C | H | N |
|---|---|---|---|
| Found | 65.27 % | 8.88 % | 1.63 % |
| Calcd. | 65.54 % | 8.31 % | 1.78 % |

(5) Color Reaction:
Positive: cerium sulfate reaction, sulfuric acid reaction, Ehrlich reaction, Dragendorff reaction and iodine vapor reaction
Negative: ferric chloride reaction, ninhydrin reaction and Molish reaction
(6) Solubility:
Soluble : methanol, ethanol, acetone, ethyl acetate, chloroform, diethyl ether, benzene and acetonitrile
Sparingly Soluble: n-hexane and petroleum ether
Insoluble: water
(7) Melting Point:
76 - 81 °C
(8) Specific Rotation:
$[\alpha]_D^{23}$ : -53° (c = 1.8, $CHCl_3$)
(9) Ultraviolet Absorption Spectrum:
end absorption
(10) Infrared Absorption Spectrum:

$$\nu_{max}^{CHCl_3}:$$

3500, 2920, 2870, 2820, 1740, 1710, 1640, 1460, 1450, 1380, 1340, 1320, 1280, 1230, 1190, 1170, 1140, 1100, 1040, 1000, 990, 960, 910 $cm^{-1}$
(11) $^{13}C$ Nuclear Magnetic Resonance Spectrum:
δ(ppm, $CDCl_3$, 100MHz):

212.16 (s) 209.71 (s) 196.10 (s)
212.17 (s), 209.73 (s), 192.94 (s),
168.89 (s) 164.55 (s) 138.85 (s)
168.69 (s), 165.65 (s), 139.57 (s),
135.39 (d) 133.61 (s) 128.69 (d)
135.25 (d), 133.33 (s), 127.98 (d),
122.41 (d) 116.51 (t), 97.04 (s)
122.60 (d), 98.54 (s),
77.71 (d), 75.10 (d) 74.71 (d),
76.42 (d),
73.52 (d) 72.67 (d) 69.58 (d)
73.46 (d), 72.24 (d), 68.61 (d),
56.92 (q) 56.43 (d), 56.16 (q)
57.48 (q), 55.89 (q),
52.78 (t) 48.57 (t) 45.14 (t)
52.71 (t), 48.27 (t), 45.12 (t),
43.74 (t) 39.74 (d) 39.15 (t)
43.89 (t), 40.29 (d), 43.80 (t),
38.66 (d), 35.12 (t), 35.01 (t)
35.34 (t),
34.46 (d) 33.05 (t), 32.50 (t)
33.70 (d), 32.36 (t),
29.89 (t) 27.53 (t) 26.06 (d)
29.94 (t), 26.13 (t), 25.98 (d),

24.31 (t) 20.96 (t) 20.17 (q)
24.45 (t), 20.73 (t), 19.38 (q),
16.07 (q) 15.76 (q) 13.56 (q)
15.83 (q), 15.71 (q), 14.00 (q),
9.48 (q)
9.73 (q),

(12) $^1$H Nuclear Magnetic Resonance Spectrum:
the chart of which being shown in Figure 2,
(13) Thin Layer Chromatography:

| Stationary Phase | Developing Solvent | Rf Value |
|---|---|---|
| silica gel plate | ethyl acetate | 0.64 |

(14) Property of the Substance:
neutral substance.

which comprises culturing a FR-901154 substance- and/or FR-901155 substance-producing strain belonging to the genus Streptomyces in a nutrient medium and recovering the FR-901154 substance and/or FR-901155 substance.

**2.** A process for the production of 1,14-dihydroxy-12-[2-(4-hydroxy-3-methoxycyclohexyl)-1-methylvinyl]-23,25-dimethoxy-13,19,21,27-tetramethyl-17-propyl-11,28-dioxa-4-azatricyclo[22.3.1.$0^{4,9}$]octacos-18-ene-2,3,10,16-tetraone (FR-901156), which comprises culturing a FR-901156 substance-producing strain belonging to the genus Streptomyces in a nutrient medium and recovering the same.

**Patentansprüche**

**1.** Ein Verfahren zur Herstellung einer aus Substanz FR-901154 und FR-901155 ausgewählten Verbindung, wobei

1) Substanz FR-901154 die folgenden physikalischen und chemischen Eigenschaften aufweist:

(1) Form und Farbe:
weißes Pulver

(2) Massenspektrum:
FAB-MS: m/z 812 (M + Na)

(3) Molekulare Formel:

$C_{43}H_{67}NO_{12}$

(4) Elementaranalyse für $C_{43}H_{67}NO_{12}.H_2O$

| | C | H | N |
|---|---|---|---|
| Gef.: | 63,80 %, | 8,81 %, | 1,79 % |
| Ber.: | 63,92 %; | 8,61 %, | 1,73 % |

(5) Farbreaktion:
Positiv: Cersulfat-Reaktion, Schwefelsäure-Reaktion, Ehrlich-Reaktion, Dragendorff-Reaktion und Ioddampf-Reaktion
Negativ: Eisenchlorid-Reaktion, Ninhydrin-Reaktion und Molish-Reaktion

(6) Löslichkeit:
löslich: Methanol, Ethanol, Aceton, Ethylacetat, Chloroform, Diethylether, Benzol und Acetonitril
kaum löslich: n-Hexan und Petrolether
unlöslich: Wasser

(7) Schmelzpunkt:
96 - 97 °C

(8) Spezifische Drehung:
$[\alpha]_D^{23}$ : -65 °C (c = 1,7 $CHCl_3$)

(9) Ultraviolett-Absorptions-Spektrum
Endabsorption

(10) Infrarot-Absorptions-Spektrum:

$$\nu_{max}^{CHCl_3}:$$

3570, 3500, 2920, 2860, 2820, 1740, 1710, 1700, 1640, 1460, 1450, 1380, 1340, 1280, 1210, 1190, 1170, 1140, 1100, 1090, 1050, 1010, 1000, 990, 960, 920 $cm^{-1}$

(11) $^{13}C$-Kernresonanz-Spektrum:
δ(ppm, $CDCl_3$, 100MHz):

{212.51 (s) {196.28 (s) {168.98 (s)
{212.44 (s), {192.78 (s), {168.71 (s),

{164.72 (s) {138.89 (s) {135.46 (d)
{165.84 (s), {139.64 (s), {135.32 (d),

{132.21 (s) {129.93 (d) {122.42 (d)
{131.78 (s), {129.50 (d), {122.60 (d),

116.58 (t), {97.00 (s) {77.47 (d)
{98.52 (s), {77.76 (d),

75.31 (d), {75.13 (d) {74.73 (d)
{76.58 (d), {74.77 (d),

{73.59 (d) {72.76 (d) {69.87 (d)
{73.56 (d), {72.18 (d), {69.02 (d),

{56.92 (q) {56.24 (q) {48.55 (t)
{57.45 (q), {56.03 (q), {48.27 (t),

{43.49 (t) {39.75 (d) {39.18 (t)
{43.86 (t), {40.22 (d), {43.86 (t),

38.95 (t), {35.19 (t) {34.90 (d)
{35.47 (t), {34.93 (d),

{34.54 (d) {32.96 (t) {32.60 (t)
{33.54 (d), {35.40 (t), {32.46 (t),

{31.94 (t) 30.83 (t), 27.58 (t),
{31.89 (t),

{26.16 (d) {24.39 (t) {21.02 (t)
{26.02 (d), {24.48 (t), {20.74 (t),

{20.31 (q) {16.14 (q) {15.84 (q)
{19.41 (q), {15.91 (q), {15.81 (q),

{13.87 (q) {9.41 (q)
{14.17 (q), {9.73 (q),

(12) $^1$H-Kernresonanz-Spektrum:
Die Abbildung ist in Figur 1 dargestellt

(13) Dünnschichtchromatographie:

| Stationäre Phase | Entwicklungslösungsmittel | Rf-Werte |
|---|---|---|
| Kieselgelplatte | Ethylacetat | 0,22 |

(14) Eigenschaft der Substanz:
Neutrale Substanz

und

2) Substanz FR-901155 die folgenden physikalischen und chemischen Eigenschaften aufweist:

(1) Form und Farbe:
weißes Pulver

(2) Massenspektrum:
FAB-MS: m/z 810 (M + Na)

(3) Molekulare Formel:

$C_{43}H_{65}NO_{12}$

(4) Elementaranalyse für $C_{43}H_{65}NO_{12}$

| | C | H | N |
|---|---|---|---|
| Gef.: | 65,27 %, | 8,88 %, | 1,63 % |
| Ber.: | 65,54 %; | 8,31 %, | 1,78 % |

(5) Farbreaktion:
Positiv: Cersulfat-Reaktion, Schwefelsäure-Reaktion, Ehrlich-Reaktion, Dragendorff-Reaktion und Ioddampf-Reaktion
Negativ: Eisenchlorid-Reaktion, Ninhydrin-Reaktion und Molish-Reaktion

(6) Löslichkeit:
löslich: Methanol, Ethanol, Aceton, Ethylacetat, Chloroform, Diethylether, Benzol und Acetonitril
kaum löslich: n-Hexan und Petrolether
unlöslich: Wasser

(7) Schmelzpunkt:
76 - 81 °C

(8) Spezifische Drehung:
$[\alpha]_D^{23}$ : -53 °C (c = 1,8 $CHCl_3$)

(9) Ultraviolett-Absorptions-Spektrum
Endabsorption

(10) Infrarot-Absorptions-Spektrum:

$$\nu_{max}^{CHCl_3}:$$

3500, 2920, 2870, 2820, 1740, 1710, 1640, 1460, 1450, 1380, 1340, 1320, 1280, 1230, 1190, 1170, 1140, 1100, 1040, 1000, 990, 960, 910 $cm^{-1}$

(11) $^{13}C$-Kernresonanz-Spektrum:
δ(ppm, $CDCl_3$, 100MHz):

| | | |
|---|---|---|
| {212.16 (s) | {209.71 (s) | {196.10 (s) |
| {212.17 (s), | {209.73 (s), | {192.94 (s), |
| {168.89 (s) | {164.55 (s) | {138.85 (s) |
| {168.69 (s), | {165.65 (s), | {139.57 (s), |
| {135.39 (d) | {133.61 (s) | {128.69 (d) |
| {135.25 (d), | {133.33 (s), | {127.98 (d), |
| {122.41 (d) | 116.51 (t), | {97.04 (s) |
| {122.60 (d), | | {98.54 (s), |
| 77.71 (d), | {75.10 (d) | 74.71 (d), |
| | {76.42 (d), | |
| {73.52 (d) | {72.67 (d) | {69.58 (d) |
| {73.46 (d), | {72.24 (d), | {68.61 (d), |
| {56.92 (q) | 56.43 (d), | {56.16 (q) |
| {57.48 (q), | | {55.89 (q), |
| {52.78 (t) | {48.57 (t) | {45.14 (t) |
| {52.71 (t), | {48.27 (t), | {45.12 (t), |
| {43.74 (t) | {39.74 (d) | {39.15 (t) |
| {43.89 (t), | {40.29 (d), | {43.80 (t), |
| 38.66 (d), | 35.12 (t), | {35.01 (t) |
| | | {35.34 (t), |
| {34.46 (d) | 33.05 (t), | {32.50 (t) |
| {33.70 (d), | | {32.36 (t), |
| {29.89 (t) | {27.53 (t) | {26.06 (d) |
| {29.94 (t), | {26.13 (t), | {25.98 (d), |
| 24.31 (t) | 20.96 (t) | 20.17 (q) |
| 24.45 (t), | 20.73 (t), | 19.38 (q), |
| 16.07 (q) | 15.76 (q) | 13.56 (q) |
| 15.83 (q), | 15.71 (q), | 14.00 (q), |
| 9.48 (q) | | |
| 9.73 (q), | | |

(12) $^1$H-Kernresonanz-Spektrum:
Die Abbildung ist in Figur 2 dargestellt

(13) Dünnschichtchromatographie:

| Stationäre Phase | Entwicklungslösungsmittel | Rf-Wert |
|---|---|---|
| Kieselgelplatte | Ethylacetat | 0,64 |

(14) Eigenschaft der Substanz:
Neutrale Substanz

das umfaßt:

Kultivieren eines zum Genus Streotomyces gehörenden Substanz FR-901154 und/oder FR-901155-produzierenden Stamms in einem Nährmedium und Isolieren der Substanz FR-901154 und/oder FR-901155.

**2.** Ein Verfahren zur Herstellung von 1,14-Dihydroxy-12-[2-(4-hydroxy-3-methoxycyclohexyl)-1-methylvinyl]-23,25-dimethoxy-13,19,21,27-tetramethyl-17-propyl-11,28-dioxa-4-azatricyclo[22.3.1.$0^{4,9}$]octacos-18-en-2,3,10,16-tetraon (FR-901156), das umfaßt:
Kultivieren eines zum Genus Streptomyces gehörenden Substanz-FR-901156-produzierenden Stamms in einem Nährmedium und Isolieren der Substanz

**Revendications**

**1.** Procédé de préparation d'un composé choisi parmi la substance FR-901154 et la substance FR-901155, dans lequel :

1) la substance FR-901154 présente les propriétés physiques et chimiques suivantes :

(1) Forme et couleur :
poudre blanche

(2) Spectre de masse :
FAB-SM: m/z 812 (M + Na)

(3) Formule brute :

$C_{43}H_{67}NO_{12}$

(4) Composition élémentaire pour $C_{43}H_{67}NO_{12}.H_2O$ :

| | C | H | N |
|---|---|---|---|
| Trouvé | 63,80 %, | 8,81 %, | 1,79 % |
| Calculé | 63,92 %, | 8,61 %, | 1,73 %. |

(5) Réactions colorées :
Positives : réaction au sulfate de cérium, réaction à l'acide sulfurique, réaction d'Ehrlich, réaction de Dragendorff et réaction à la vapeur d'iode;
négatives : réaction au chlorure ferrique, réaction à la ninhydrine et réaction de Molish

(6) Solubilité :
Soluble : méthanol, éthanol, acétone, acétate d'éthyle, chloroforme, éther diéthylique, benzène et acétonitrile
Peu soluble : n-hexane et éther de pétrole
Insoluble : eau

(7) Point de fusion :
96 - 97 ° C.

(8) Pouvoir rotatoire spécifique :
$[\alpha]_D^{23}$ : -65 ° C (c = 1,7, $CHCl_3$)

(9) Spectre d'absorption d'UV :
absorption finale

(10) Spectre d'absorption d'Infra-rouge

$$\nu_{max}^{CHCl_3} :$$

3570, 3500, 2920, 2860, 2820, 1740, 1710, 1700, 1640, 1460, 1450, 1380, 1340, 1280, 1210, 1190, 1170, 1140, 1100, 1090, 1050, 1010, 1000, 990, 960, 920 $cm^{-1}$.

(11) Spectre de résonance magnétique nucléaire $^{13}C$:

$\delta$ (ppm, $CDCl_3$, 100 MHz) :

| | | |
|---|---|---|
| {212,51 (s)<br>212,44 (s), | {196,28 (s)<br>192,78 (s), | {168,98 (s)<br>168,71 (s), |
| {164,72 (s)<br>165,84 (s), | {138,89 (s)<br>139,64 (s), | {135,46 (d)<br>135,32 (d), |
| {132,21 (s)<br>131,78 (s), | {129,93 (d)<br>129,50 (d), | {122,42 (d)<br>122,60 (d), |
| 116,58 (t), | {97,00 (s)<br>98,52 (s), | {77,47 (d)<br>77,76 (d), |
| 75,31 (d), | {75,13 (d)<br>76,58 (d), | {74,73 (d)<br>74,77 (d), |
| {73,59 (d)<br>73,56 (d), | {72,76 (d)<br>72,18 (d), | {69,87 (d)<br>69,02 (d), |
| {56,92 (q)<br>57,45 (q), | {56,24 (q)<br>56,03 (q), | {48,55 (t)<br>48,27 (t), |
| {43,49 (t)<br>43,86 (t), | {39,75 (d)<br>40,22 (d), | {39,18 (t)<br>43,86 (t), |
| 38,95 (t), | {35,19 (t)<br>35,47 (t), | {34,90 (d)<br>34,93 (d), |
| {34,54 (d)<br>33,54 (d), | {32,96 (t)<br>35,40 (t), | {32,60 (t)<br>32,46 (t), |
| {31,94 (t)<br>31,89 (t), | 30,83 (t), | 27,58 (t), |
| {26,16 (d)<br>26,02 (d), | {24,39 (t)<br>24,48 (t), | {21,02 (t)<br>20,74 (t), |
| {20,31 (q)<br>19,41 (q), | {16,14 (q)<br>15,91 (q), | {15,84 (q)<br>15,81 (q), |
| {13,87 (q)<br>14,17 (q), | {9,41 (q)<br>9,73 (q), | |

(12) Spectre dc résonance magnétique nucléaire $^{1}H$:

son tracé est reproduit dans la figure 1

(13) Chromatographie sur couche mince :

| Phase stationnaire | Solvant de développement | Rf |
|---|---|---|
| Plaque de gel de silice | acétate d'éthyle | 0,22 |

(14) Propriété de la substance :
substance neutre

et

2) La substance FR-901155 présente les propriétés physiques et chimiques suivantes :

(1) Forme et couleur :
poudre blanche

(2) Spectre de masse :
FAB-SM: m/z 810 (M + Na)

(3) Formule brute :

$C_{43}H_{65}NO_{12}$

(4) Composition élémentaire pour $C_{43}H_{65}NO_{12}$ :

| | C | H | N |
|---|---|---|---|
| Trouvé | 65,27 %, | 8,88 %, | 1,63 % |
| Calculé | 65,54 %, | 8,31 %, | 1,78 %. |

(5) Réactions colorées :

Positives : reaction au sulfate de cérium, réaction à l'acide sulfurique, réaction d'Ehrlich, réaction de Dragendorff et réaction à la vapeur d'iode;

négatives : réaction au chlorure ferrique, réaction à la ninhydrine et réaction de Molish

(6) Solubilité :

Soluble : méthanol, éthanol, acétone, acétate d'éthyle, chloroforme, éther diéthylique, benzène et acétonitrile

Peu soluble : n-hexane et éther de pétrole

Insoluble : eau

(7) Point de fusion :
76 - 81 °C.

(8) Pouvoir rotatoire spécifique :
$[\alpha]_D^{23}$ : -53 °C (c = 1,8, $CHCl_3$)

(9) Spectre d'absorption d'UV :
absorption finale

(10) Spectre d'absorption d'Infra-rouge

$$\nu_{max}^{CHCl_3} :$$

3500, 2920, 2870, 2820, 1740, 1710, 1640, 1460, 1450, 1380, 1340, 1320, 1280, 1230, 1190, 1170, 1140, 1100, 1040, 1000, 990, 960, 910 $cm^{-1}$.

(11) Spectre de résonance magnétique nucléaire $^{13}C$:
$\delta$ (ppm $CDCl_3$, 100 MHz) :

| | | |
|---|---|---|
| 212,16 (s) | 209,71 (s) | 196,10 (s) |
| 212,17 (s), | 209,73 (s), | 192,94 (s), |
| 168,89 (s) | 164,55 (s) | 138,85 (s) |
| 168,69 (s), | 165,65 (s), | 139,57 (s), |
| 135,39 (d) | 133,61 (s) | 128,69 (d) |
| 135,25 (d), | 133,33 (s), | 127,98 (d), |
| 122,41 (d) | 116,51 (t), | 97,04 (s) |
| 122,60 (d), | | 98,54 (s), |
| 77,71 (d), | 75,10 (d) | 74,71 (d), |
| | 76,42 (d), | |
| 73,52 (d) | 72,67 (d) | 69,58 (d) |
| 73,46 (d), | 72,24 (d), | 68,61 (d), |
| 56,92 (q) | 56,43 (d), | 56,16 (q) |
| 57,48 (q), | | 55,89 (q), |
| 52,78 (t) | 48,57 (t) | 45,14 (t) |
| 52,71 (t), | 48,27 (t), | 45,12 (t), |
| 43,74 (t) | 39,74 (d) | 39,15 (t) |
| 43,89 (t), | 40,29 (d), | 43,80 (t), |
| 38.66 (d), | 35,12 (t), | 35,01 (t) |
| | | 35,34 (t), |
| 34,46 (d) | 33,05 (t), | 32,50 (t) |
| 33,70 (d), | | 32,36 (t), |
| 29,89 (t) | 27,53 (t) | 26.06 (d) |
| 29.94 (t), | 26,13 (t), | 25,98 (d), |
| 24,31 (t) | 20,96 (t) | 20,17 (q) |
| 24,45 (t), | 20,73 (t), | 19,38 (q), |
| 16,07 (q) | 15,76 (q) | 13,56 (q) |
| 15,83 (q), | 15,71 (q), | 14,00 (q), |
| 9,48 (q) | | |
| 9,73 (q), | | |

(12) Spectre de résonance magnétique nucléaire $^1H$:
son tracé est représenté dans la figure 2

(13) Chromatographie sur couche mince :

| Phase stationnaire | Solvant de développement | Rf |
|---|---|---|
| Plaque de gel de silice | acétate d'éthyle | 0,64 |

(14) Propriété de la substance :
substance neutre,

qui comprend le fait de cultiver une souche produisant une substance FR-901154 et/ou une substance FR-901155 appartenant au genre Streptomyces dans un milieu nutritif et de récupérer la substance FR-901154 et/ou la substance FR-901155.

**2.** Procédé pour la production du 1,14-dihydroxy-12-[2-(4-hydroxy-3-méthoxycyclohexyl)-1-méthylvinyl]-23,25-diméthoxy-13,19-21,27-tétraméthyl-17-propyl-11,28-dioxa-4-azatricyclo[23.3.1.$0^{4,9}$]octacos-18-ène-2,3,10,16-tétraone (FR-901156), qui comprend le fait de cultiver une souche produisant une substance FR-901156 appartenant au genre Streptomyces dans un milieu nutritif et de récupérer la substance.

Figure 1

$^{1}H$ Nuclear Resonance Spectrum of FR-901154 Substance (400 MHz, in $CDCl_3$)

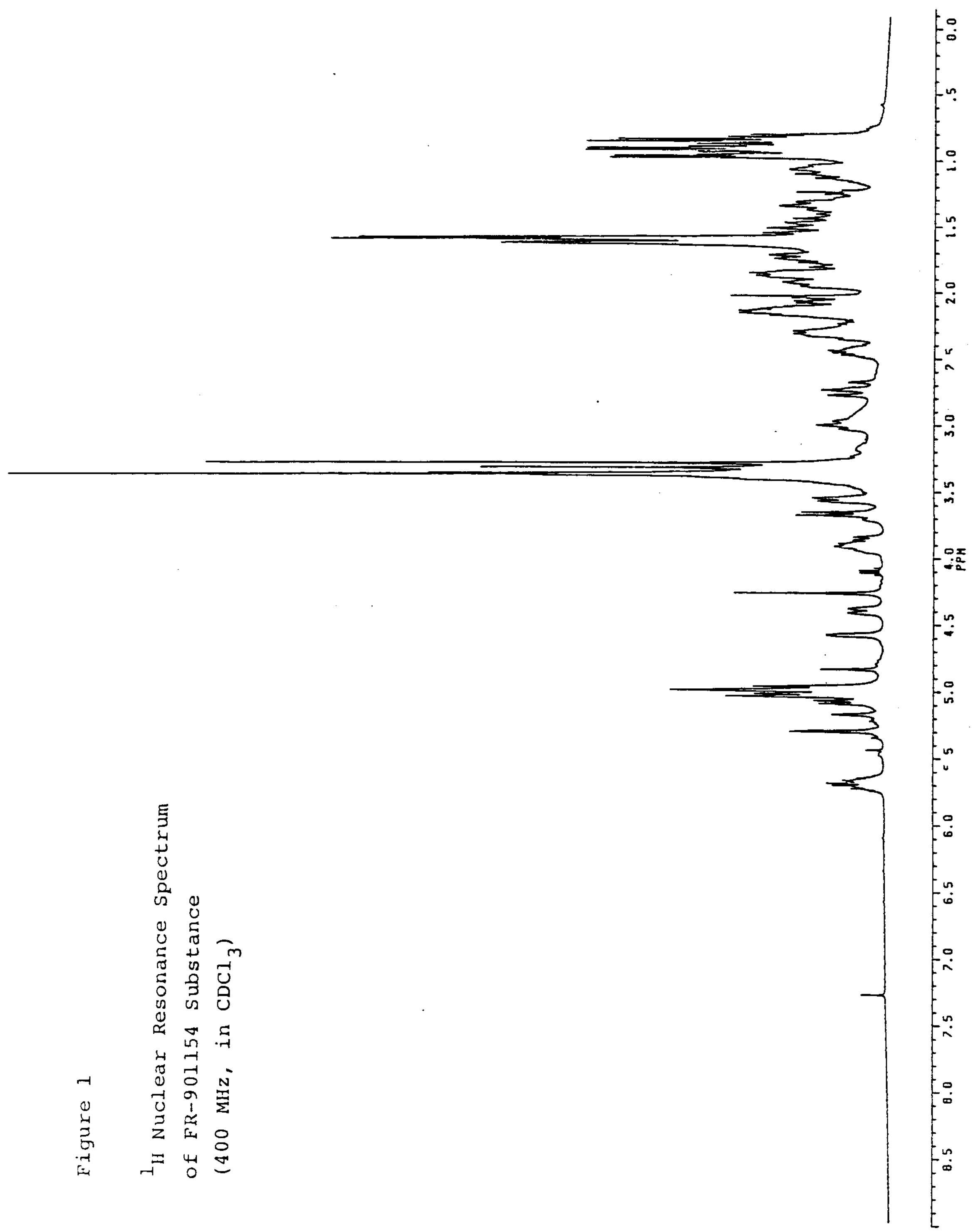

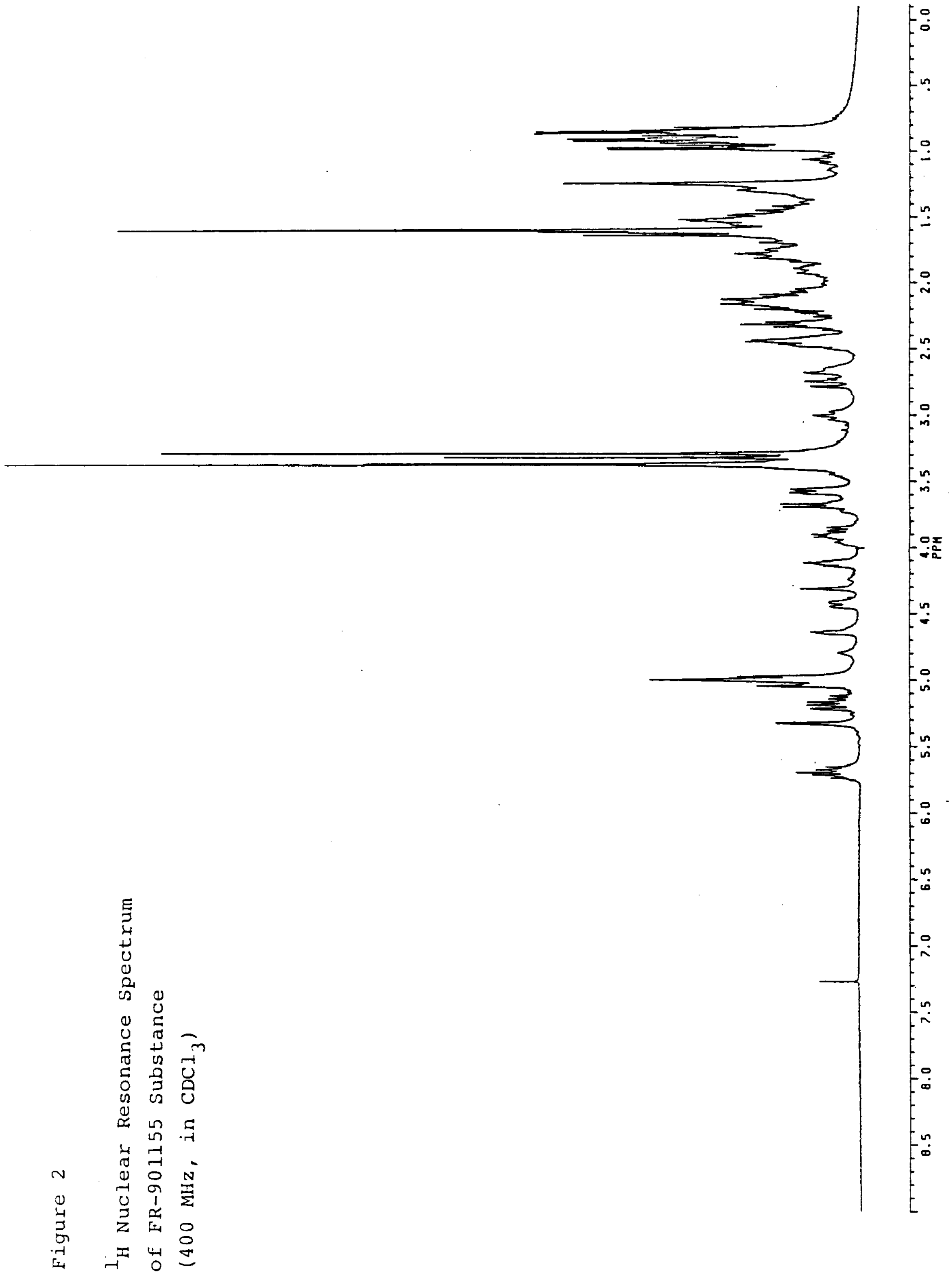


Figure 2

$^1H$ Nuclear Resonance Spectrum of FR-901155 Substance (400 MHz, in $CDCl_3$)

Figure 3

$^1H$ Nuclear Resonance Spectrum of FR-901156 Substance (400 MHz, in $CDCl_3$)